(19) Europäisches Patentamt
European Patent Office
Office européen des brevets

(11) EP 0 507 672 B1

(12) FASCICULE DE BREVET EUROPEEN

(45) Date de publication et mention de la délivrance du brevet: **04.06.1997 Bulletin 1997/23**

(51) Int Cl.[6]: **C07D 405/12**, C07D 295/12, A61K 31/165, C07D 211/14, C07D 211/22

(21) Numéro de dépôt: **92400897.2**

(22) Date de dépôt: **31.03.1992**

# (54) Dérivés de N-cyclohexyl benzamides, leurs préparations et leurs applications en thérapeutique

N-Cyclohexyl Benzamide Derivate, ihre Herstellungen und therapeutischen Anwendungen

N-cyclohexyl benzamides derivatives, preparations and therapeutic applications thereof

(84) Etats contractants désignés:
**AT BE CH DE DK ES FR GB GR IT LI LU NL PT SE**

(30) Priorité: **02.04.1991 FR 9103959**

(43) Date de publication de la demande:
**07.10.1992 Bulletin 1992/41**

(73) Titulaire: **LABORATOIRES JACQUES LOGEAIS Société dite:**
**92130 Issy-Les-Moulineaux (FR)**

(72) Inventeurs:
- **Christinaki, Hélène**
  **F-92360 Meudon-la-Foret (FR)**
- **Bouyssou, Thierry**
  **F-78370 Plaisir (FR)**
- **Pairet, Michel**
  **F-78990 Elancourt (FR)**
- **Renaud, Alain**
  **F-92500 Rueil Malmaison (FR)**

(74) Mandataire: **Le Guen, Gérard et al**
**CABINET LAVOIX**
**2, place d'Estienne d'Orves**
**75441 Paris Cédex 09 (FR)**

(56) Documents cités:

| | |
|---|---|
| **EP-A- 0 076 530** | **EP-A- 0 094 742** |
| **EP-A- 0 222 533** | **EP-A- 0 389 037** |
| **EP-A- 0 445 862** | **FR-A- 2 264 530** |
| **FR-A- 2 370 722** | **GB-A- 1 015 921** |

- **Eur. J. Clin. Pharmacol. (1990); 38:161-164**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 507 672 B1

Printed by Jouve, 75001 PARIS (FR)

## Description

La présente invention concerne de nouveaux dérivés de N-cyclohexyl benzamides qui constituent de puissants stimulants de la motricité gastro-intestinale.

De nombreuses orthopamides sont connues pour différentes propriétés pharmacologiques, comme par exemple les neuroleptiques dérivés du Sulpiride (US-A-3,342,826) ou les anti-émétiques et stimulants de la motricité gastrique tels que le Métoclopramide (BE-A-620,543). Le dernier produit est connu pour agir sur les voies dopaminergiques du système nerveux central.

Des composés de la famille des benzamides plus récents tels que le Cisapride (EP-A-076,530) ou le Renzapride (EP-A-094,742) sont connus pour leur activité sur la motricité digestive en l'absence d'effets antidopaminergiques centraux.

Par ailleurs, des composés répondant à la formule générale

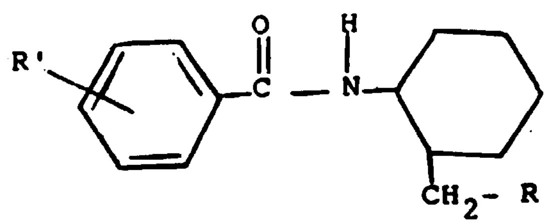

dans laquelle R est un groupe amino tertiaire, notamment un groupe pipéridino et R' est un groupe alkoxy en para ou en meta sur le noyau benzamide, sont connus pour leur propriété anticonvulsivante (GB-A- 1,015,921).

Nous avons maintenant découvert que les composés représentés par la formule générale (I) définis ci-après et qui diffèrent des composés précédents notamment en ce qu'ils comportent un noyau aromatique polysubstitué et en particulier un groupe alkoxy en ortho, sont de puissants stimulants de la motricité gastrointestinale et dépourvus d'activité antagoniste dopaminergique centrale, alors que les composés décrits ne possédant pas cette substitution ne présentent pas une telle activité. A titre d'exemple un composé A du type décrit dans GB-A-1,015,921 répondant à la formule générale I mais avec $R_1 = R_3 = R_4 = H$ et $R_2$ = 4-pentyloxy, ne montre aucune activité sur la motricité intestinale à la dose la plus élevée utilisée pour tester les composés selon l'invention.

La présente invention a ainsi pour objet de nouveaux benzamides répondant à la formule générale (I)

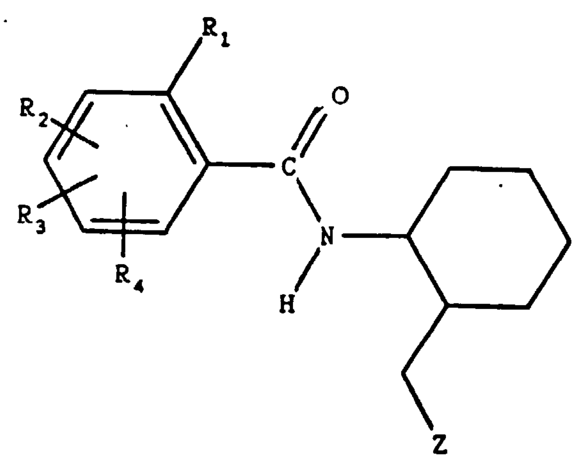

(I)

dans laquelle :

$R_1$ est choisi parmi les groupes alkoxy en $C_1$-$C_4$, méthoxy alkoxy en $C_1$-$C_3$, alkenyloxy en $C_3$-$C_4$ et cyclo alkyloxy en $C_5$-$C_6$,

ou $R_1$ et $R_2$, en position 3, forment ensemble et avec le noyau aromatique sur lequel ils sont fixés un cycle 2,3-dihydrobenzofuranne,

$R_2$, $R_3$, $R_4$ sont choisis indépendamment l'un de l'autre parmi un atome d'hydrogène, un atome d'halogène et les groupes hydroxy, alkoxy en $C_1$-$C_3$, amino, (alkyl en $C_1$-$C_3$) amino, di(alkyl en $C_1$-$C_3$) amino et (alkyl en $C_1$-$C_3$) carbonyl amino,

Z est choisi parmi les groupes pipéridinyle et pipéridinyle substitué en 4 par un groupe hydroxy, alkoxy en $C_1$-$C_3$, hydroxyéthyle, (alkoxy en $C_1$-$C_3$)éthyle, (alkoxy en $C_1$-$C_3$) méthyle, un groupe alkyle en $C_1$-$C_4$, ou deux groupes alkyle en $C_1$-$C_3$,

leurs N-oxydes, notamment ceux dans lesquels le groupe N-oxyde est porté par Z,

et leurs sels d'addition avec des acides pharmaceutiquement acceptables.

La présente invention englobe les stéréosimères de configuration relative cis et de configuration relative trans, formés par les substituants du cycle cyclohexyle, ainsi que les énantiomères correspondants.

Dans la présente invention,par groupe alkyle ou alkoxy, on désigne aussi bien des groupes linéaires, ramifiés ou présentant une partie cyclique.

Dans la présente invention, on désigne par "sels pharmaceutiquement acceptables" sels d'addition avec des acides qui donnent les propriétés biologiques des composés sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; les sels métalliques acides, tels que l'orthophosphate disodique et le sulfate monopotassique, et des acides organiques, tels que l'acide formique, l'acide acétique, l'acide propionique, l'acide glycolique, l'acide oxalique, l'acide fumarique, l'acide lactique, l'acide succinique, l'acide tartrique, l'acide malique et l'acide pamoïque.

Les composés de formule I préférés sont ceux qui possèdent sur le noyau aromatique un substituant alcoxy en 2, en particulier un groupe méthoxy, éthoxy ou cyclopropylméthoxy, un substituant amino en 4 et chloro en 5.

Parmi ces benzamides, on préfère plus particulièrement les composés de formule (I) dans laquelle Z est un groupe pipéridino, de préférence substitué en 4 par un ou deux groupes méthyle ou par un groupe hydroxy.

Les composés préférés ont donc la structure (IV) ou (V).

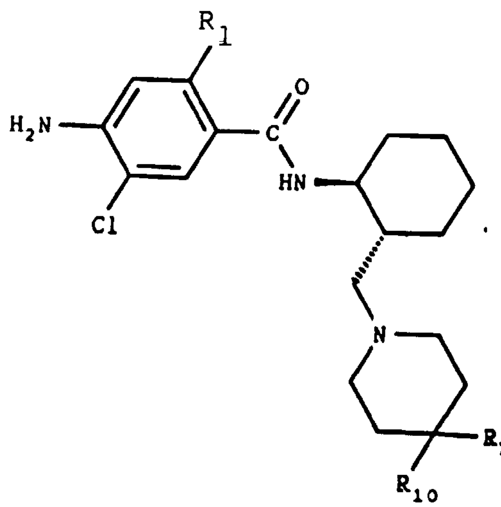

(IV)

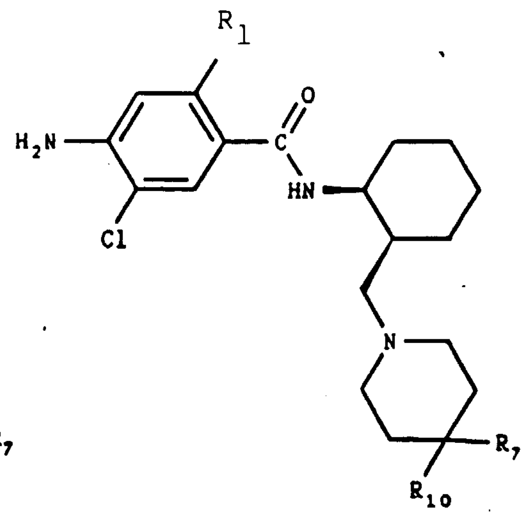

(V)

dans lesquelles $R_7 = CH_3$, $R_{10} = H$ ou $R_7 = CH_3 = R_{10}$ ou $R_7 = OH$, $R_{10} = H$ et $R_1 = OCH_3$, $OC_2H_5$ ou cyclopropylméthoxy.

Les composés de formule (I) peuvent être obtenus par condensation entre un acide benzoïque de formule VI où $R_1$, $R_2$, $R_3$, $R_4$ ont les mêmes définitions que précédemment, et une cyclohexyl amine de formule (VII) de configuration cis ou trans avec Z ayant les mêmes définitions que dans la formule (I), selon le schéma

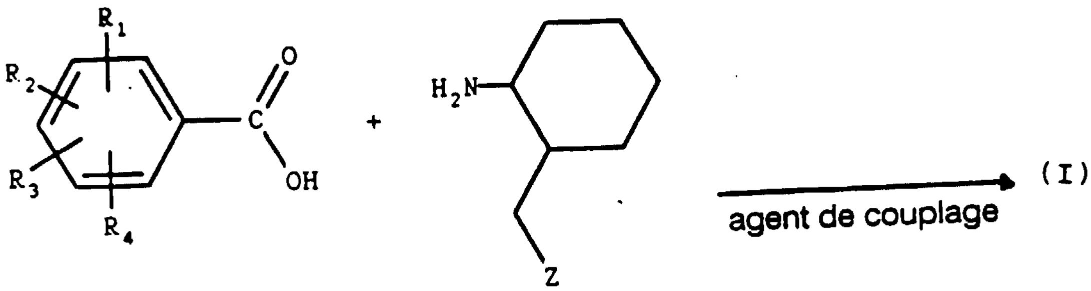


(VI) (VII)

L'agent de couplage peut être le carbonyldiimidazole ou le dicyclohexyl carbodiimide utilisé dans des solvants tels que le chlorure de méthylène ou le tétrahydrofuranne ou même la pyridine.

Dans le cas où l'acide benzoïque de formule VI ne porte pas de fonction basique amino ou alkylamino, il peut être activé sous forme de chlorure d'acide par action du chlorure de thionyle dans un solvant tel que le toluène, avant réaction avec l'amine de formule VII.

L'amine de formule VII peut être obtenu par plusieurs voies différentes conduisant soit au diastéréoisomère de configuration cis de formule VII', soit au diastéréoisomère de configuration trans de formule VII".

Voie 1

Le composé de formule VII' peut être obtenu par réaction de l'amine cis de formule VIII' à l'aide d'hydrure tel que l'hydrure d'aluminium et de lithium dans le tétrahydrofuranne

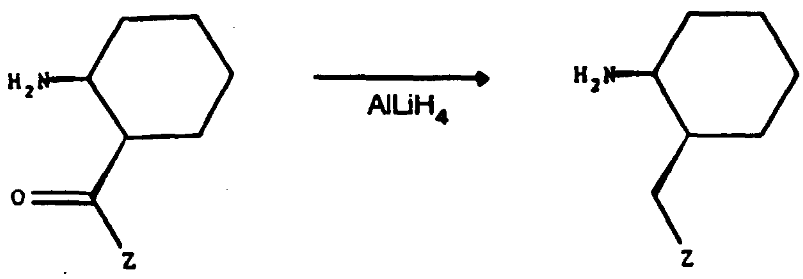


(VIII') (VII')

De même le composé de formule VII" peut être obtenu par réduction de l'amide de formule VIII"

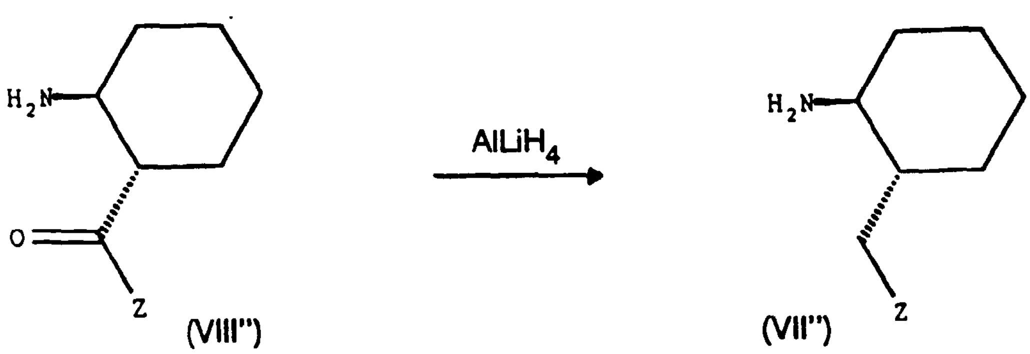


(VIII") (VII")

Les composés VIII sont obtenus en trois étapes à partir des β-amino acides correspondants suivant le schéma suivant :

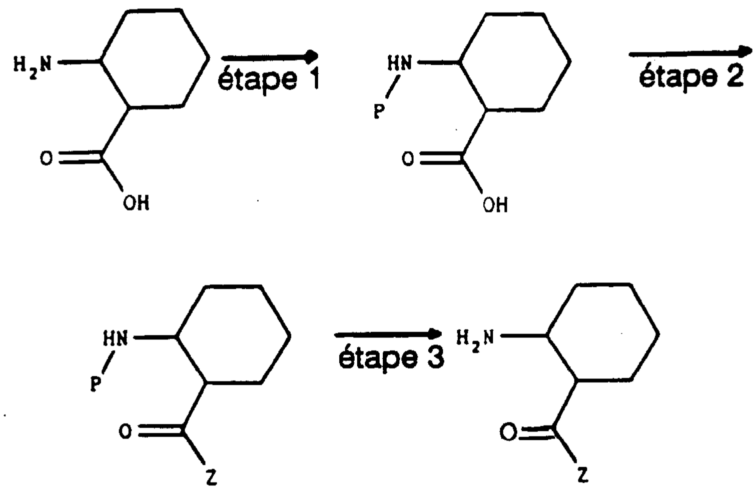


L'étape 1 consiste à protéger l'amine primaire par un groupe acyle tel que trifluoracétyle comme déjà décrit dans la littérature (Act. Chim. Acad. Scient. Hung 99 (2), 175-192 (1979)) ou uréthane tel que benzyloxycarbonyle ou t-butoxycarbonyle ou tout autre groupe communément utilisé dans la synthèse des peptides.

L'étape 2 consiste à condenser le groupe Z soit par une des méthodes décrites pour la préparation de (I), soit même par la formation intermédiaire d'un anhydre mixte, obtenu à partir de chloroformiates.

L'étape 3 consiste à déprotéger la fonction amine, en employant un milieu alcalin dans le cas d'un trifluoroacétyle, une hydrogénation catalytique dans le cas d'un groupe benzyloxycarbonyle, ou l'acide trifluoroacétique dans le chlorure de méthylène dans le cas d'un groupe t-butoxycarbonyle.

Les β-amino acides de départ de configuration relative cis ou trans sont obtenus par les méthodes déjà décrites dans la littérature.

La préparation des composés VII optiquement purs peut être effectuée par cristallisation stéréosélective des β-amino-acides N-protégés, par exemple N-benzoylés, sous forme d'un sel obtenu à l'aide d'une amine optiquement active, telle que la cinchonidine. Un tel dédoublement est décrit dans J. Chem. Soc. (1970), 1597-1600. Le β-amino-acide optiquement pur est ensuite converti en diamine VII par l'enchaînement des réactions suivantes :

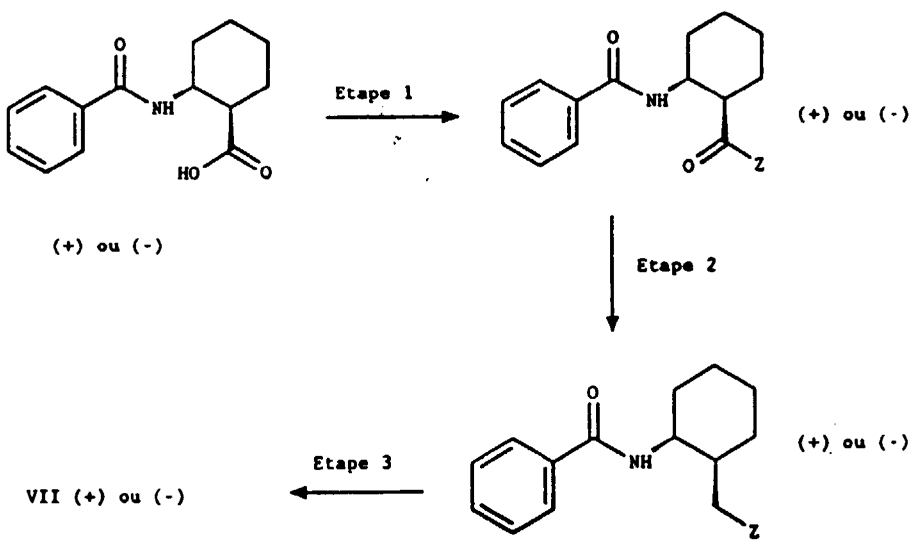


L'étape 1 consiste à condenser le groupe Z par les méthodes déjà décrites. L'étape 2 consiste à réduire sélectivement la fonction amide tertiaire par un hydrure tel que $AlLiH_4$. L'étape 3 consiste à hydrolyser le groupe benzoyle en milieu acide aqueux. La suite des réactions s'effectue sur l'un ou l'autre des énantiomères.

Une autre méthode d'obtention de composés optiquement purs consiste à effectuer une hydrolyse stéréosélective du 1,2-cyclohex-4-ènedicarboxylate de diméthyle à l'aide d'une lipase telle que la lipase de foie de porc. On obtient

après réarrangement de Curtius effectué sur l'acide-ester des β-amino-acides optiquement purs.

Une telle méthodologie est décrite dans Tetrahedron Letters (1984), 25, 2557-2560. La double liaison du cyclohexène est réduite par hydrogénation en présence d'un catalyseur tel que le palladium sur charbon. Cette étape peut s'effectuer à tout moment dans la suite des opérations en fonction des groupes protecteurs utilisés. Ces β-amino-acides sont convertis en diamine VII par les méthodes décrites précédemment.

Voie 2

Les composés de formule VII peuvent également être obtenus par une réaction de Mannich sur la cyclohexanone, suivie d'une réaction d'amination réductive passant soit par l'oxime, soit par une imine telle que celle obtenue avec la benzylamine, suivant le schéma :

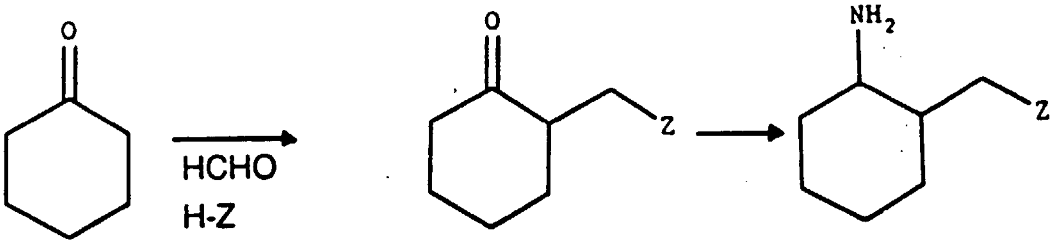


(IX) (VII)

Dans ce cas, un mélange de composé cis et trans est obtenu dont la proportion varie avec l'agent réducteur utilisé à la dernière étape. A titre d'exemple l'oxime obtenue à partir de la cétone de formule (IX) fournit majoritairement une amine trans VII" par réduction à l'aide de sodium dans l'alcool isoamylique et majoritairement une amine cis VII' après réduction par l'hydrure AlLiH4.

Voie 3

Les composés de formule générale VII" peuvent aussi être obtenus à partir de la cétone IX par réduction à l'aide par exemple de K sélectride (de formule K(s-Bu)-3BH) dans le tétrahydrofuranne.

L'alcool cis obtenu est ensuite activé,-par exemple par l'intermédiaire d'un mésylate, puis substitué par une fonction capable de générer une amine primaire telle que l'azide ou le phtalimide. Le précurseur est ensuite converti en amine par les méthodes connues dans la littérature. Le schéma réactionnel est le suivant :

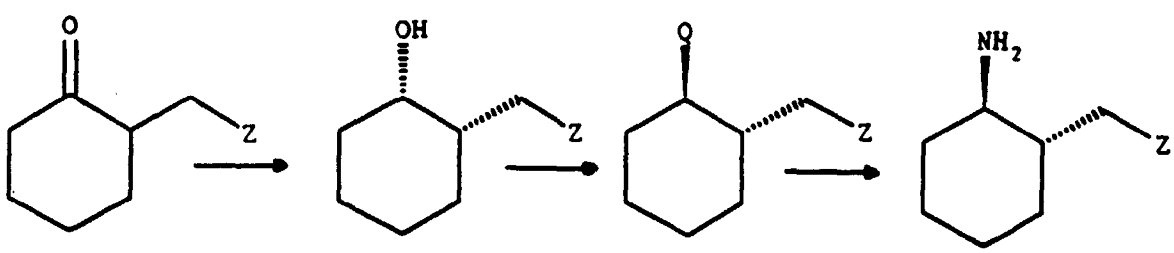

où Q = $N_3$, Phtalimide.

Les exemples suivants illustrent la préparation des composés de formule 1.

Exemple 1

N-[cis 2-(4-méthyl pipéridinométhyl) cyclohexyl]-4-amino-5-chloro-2-méthoxybenzamide.

A une solution d'acide 4-amino-5-chloro-2-méthoxy benzoïque (3,67 g; 18,2 mmoles) dans du THF (50 ml), est ajouté du carbonyldiimidazole (2,95 g; 18,2 mmoles).

Après une agitation d'une heure à température ambiante, la cis 2-(4-méthyl pipéridinométhyl) cyclohexylamine (3,83 g; 18,2 mmoles) est ajoutée en solution dans du THF (15 ml). Le mélange est agité une nuit à température

ambiante. Le mélange est évaporé sous pression réduite. Le résidu est repris par le chlorure de méthylène, lavé par une solution aqueuse de bicarbonate de sodium à 8% puis par de l'eau.

La phase organique est séchée sur sulfate de sodium et le solvant chassé sous pression réduite pour donner des cristaux (5,87 g ; 83 %) qui sont recristallisés dans un mélange éther isopropylique/acétone (8/2).

Point de fusion141-142° C.
IR : (C=O) : 1638 $cm^{-1}$ ($CHCl_3$)

Exemple 2

N-[cis 2-(4-méthyl pipéridinométhyl) cyclohexyl]-4-amino-5-chloro-2-méthoxy benzamide, chlorhydrate, hydrate.

Le produit obtenu à l'exemple précédent (4,56 g) est dissous dans l'éthanol absolu (15 ml) et traité par une solution d'acide chlorhydrique 4 N dans l'éther éthylique (4,3 ml). Le milieu est concentré et le chlorhydrate est cristallisé sous forme d'hydrate (5,4 % d'eau) par addition d'eau (30 ml).

Point de fusion : 58 - 145° C (décomposition).
IR : (C=O) : 1627 $cm^{-1}$ (KBr).

Exemple 3

N-[trans 2-(4-méthyl pipéridinométhyl) cyclohexyl]-4-amino-5-chloro-2-méthoxy benzamide, dichlorhydrate.

Le produit est préparé comme dans l'exemple 1 en utilisant la trans 2-(4-méthyl pipéridinométhyl) cyclohexylamine. Le produit est purifié par chromatographie sur colonne de silice en éluant par un système chlorure de méthylène/acétate d'éthyle/méthanol/ammoniaque (85/15/5/0.3). L'huile obtenue est traitée par de l'éther chlorhydrique, le solvant évaporé et le résidu repris par l'acétone pour fournir des cristaux.

F : 182-184° C
IR : (C=O) : 1646 $cm^{-1}$ (KBr).

Exemple 4

N-[cis 2-(4,4-diméthyl pipéridinométhyl) cyclohexyl]-4-amino-5-chloro-2-méthoxy benzamide chlorhydrate, hemi-hydrate.

Le produit est préparé comme à l'exemple 3 à partir de la cis 2-(4,4-diméthyl pipéridinométhyl) cyclohexylamine.

F : 132-153° C (dec.)
IR : (C=O) : 1617 $cm^{-1}$ ($CHCl_3$).

Les autres composés sont obtenus par des méthodes identiques en utilisant l'acide benzoïque, et le dérivé de cyclohexylamine correspondant. Pour l'acide 3-chloro-2,4,6-triméthoxy benzoïque le chlorure d'acide est utilisé comme espèce activée intermédiaire. Les caractéristiques de ces composés sont résumées dans le tableau I.

## TABLEAU I

| Exemples N° | $R_1, R_2, R_3, R_4$ | Z (configuration) | Sel | Fusion °C | Purification | IR ($cm^{-1}$) |
|---|---|---|---|---|---|---|
| 5 | 4-amino-5 chloro-2 méthoxy | pipéridino (cis) | base | 153-170 (dec) | Chromatographie $SiO_2$ | 1637,1617 ($CHCl_3$) |
| 6 | " | " (trans) | base | 100-113 | " | 1634, 1616 ($CHCl_3$) |
| 7 | " | 4-éthyl pipéridino (cis) | HCl | 162-173 (dec) | Acétone/ Acétonitrile | 1616 ($CHCl_3$) |
| 8 | " | 4-propyl pipéridino (cis) | HCl | 192 (dec) | EtOH/$H_2O$ | 1616 ($CHCl_3$) |
| 9 | " | 4-t butyl pipéridino (cis) | HCl, $H_2O$ | 140-167 (dec) | $H_2O$ | 1623 (KBr) |
| 10 | " | 3,5-diméthyl pipéridino (cis) | HCl, $H_2O$ | 142-152 (dec) | $H_2O$ | 1628 (KBr) |
| 11 | " | 4-méthyl-4-éthyl pipéridino (cis) | HCl, 1,5 $H_2O$ | 185-190 | $H_2O$ | 1623 (KBr) |
| 12 | " | 4-méthoxyéthyl pipéridino (cis) | base | 98-106 | éther de pétrole/Acétate d'éthyle | 1637-1617 ($CHCl_3$) |
| 13 | 3-chloro-2,4,6 triméthoxy | pipéridino (cis) | HCl | 220-230 | Acétone | 1623 (KBr) |
| 14 | " | pipéridino (trans) | base | 212-214 (dec) | Chromatographie $SiO_2$ | 1648 ($CHCl_3$) |
| 15 | " | 4-méthyl pipéridino (cis) | HCl | 228-236 | Chromatographie $Al_2O_3$ | 1685-1596 (large) (KBr) |
| 16 | " | 4-méthyl pipéridino (trans) | base | 120-121 | Chromatographie $SiO_2$ | 1648 ($CHCl_3$) |

## Suite du tableau I

| N° | $R_1,R_2,R_3,R_4$ | Z (Configuration) | Sel | Fusion °C | Purification | IR ($cm^{-1}$) | $[\alpha]_D^{20°}$ = (C %) |
|---|---|---|---|---|---|---|---|
| 17 | 4-amino-5-chloro-2 éthoxy | 4-méthyl pipéridine (cis) | base | 137,5-138,5 | Acétone/éther éthylique | 1636-1616 ($CHCl_3$) | / |
| 18 | 4-amino-5-chloro-2-cyclopropyl méthoxy | " | HCl | 220-278 dec | $H_2O$ | 1616 (KBr) | / |
| 19 | 4-amino-5-chloro-2 propyloxy | " | HCl | 213-241 dec | $H_2O$ | 1615 (KBr) | / |
| 20 | 4-amino-5-chloro-2 ipropyloxy | " | HCl | 126-135 dec | $H_2O$ | 1627 (KBr) | / |
| 21 | 4-amino-5-chloro-2-allyloxy | " | HCl | 144-147 | $H_2O$ | 1618 (KBr) | / |
| 22 | 4-amino-5-chloro-2-méthoxy éthoxy | " | HCl | 191-258 dec | $H_2O$ | 1614 (KBr) | / |
| 23 | 4-amino-5-chloro-2-méthoxy | " (R,R) | HCl | 188-224 dec | $H_2O$ | 1624 (KBr) | -97,8 (1, MeOH) |
| 24 | 4-amino-5-chloro-2-méthoxy | " (S,S) | HCl | 187-223 dec | $H_2O$ | 1627 (KBr) | +98,3 (1, MeOH) |
| 25 | 4-amino-5-chloro-2-cyclo-propyl méthoxy | " (R,R) | base | 151-153 | $CH_2Cl_2/Et_2O$ | 1636-1617 ($CHCl_3$) | -65,8 (1, MeOH) |
| 26 | 4-amino-5-chloro-2-cyclo-pentyloxy | 4-méthyl pipéridine (cis) | HCl | 239-258 dec | $H_2O$ | 1624 (KBr) | |
| 27 | 4-amino-5-chloro-2-isobutyloxy | 4-méthyl pipéridine (cis) | HCl | 136-143 dec | $H_2O$ | 1627 (KBr) | / |

| N° | $R_1,R_2,R_3,R_4$ | Z (Configuration) | Sel | Fusion °C | Purification | IR ($cm^{-1}$) |
|---|---|---|---|---|---|---|
| 28 | 4-amino-5-chloro-2-méthoxy | 4-hydroxy pipéridino (cis) | HCl | 254-263 dec | Acétone/eau | 1629 (KBr) |
| 29 | " | 4-éthoxy pipéridino (cis) | HCl | 160-166 | $H_2O$ | 1618 (KBr) |
| 30 | " | 4-méthoxy méthyl pipéridino (cis) | base | 148-149 | Acétone/éther de pétrole | 1638-1617 ($CHCl_3$) |
| 31 | " | 4-hydroxy éthyl pipéridino (cis) | HCl | 138-143 | Acétone | 1629 (KBr) |

**Tableau I (suite)**

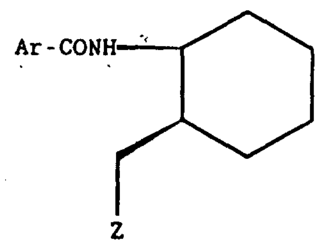

| N° | Ar | Z (Configuration) | Sel | Fusion °C | Purification | IR ($cm^{-1}$) |
|---|---|---|---|---|---|---|
| 32 | 7-(2,3-dihydro)benzofuranyl | 4-méthyl pipéridino (cis) | HCl | 177-181 | Acétone | 1643 (KBr) |
| 33 | 7-(2,3-dihydro)benzofuranyl | 4-hydroxy pipéridino (cis) | HCl | 233-238 dec | $H_2O$ | 1651 ($CHCl_3$) |

Les exemples suivants illustrent la préparation des cyclohexylamines de formule VII.

EXEMPLE A

cis 2-(4-méthyl pipéridinométhyl) cyclohexylamine.

a) 1-[cis(2-trifluoroacétamido cyclohexyl carbonyl)]-4-méthyl pipéridine.

Le chlorure de l'acide cis 2-trifluoroacétamido cyclohexanecarboxylique (8,61 g ; 33 mmoles) en solution dans du benzène (50 ml) est traité goutte à goutte par la 4-méthyl pipéridine (8,3 g ; 83 mmoles) en solution dans le benzène (20 ml). Le milieu réactionnel est maintenu 1h30 à température ambiante avant évaporation du solvant. Le résidu est repris par du chloroforme, la phase organique lavée à l'eau, séchée sur sulfate de sodium puis évaporée sous pression réduite. Le produit obtenu est recristallisé dans un mélange benzène/éther de pétrole.

F : 135,5° C
IR : (C=O) : 1718 - 1621,5 $cm^{-1}$ ($CHCl_3$).

b) 1-[cis(2-aminocyclohexyl carbonyl]-4-méthyl pipéridine.

La 1-[cis(2-trifluoroacétamido cyclohexyl) carbonyl]-4-méthyl pipéridine (8,8 g ; 0,027 mole) est dissoute dans l'éthanol à 95 % (250 ml), puis une solution de soude 1 N dans l'éthanol à 95 % (50 ml ; 0,05 mole) est ajoutée.

Le milieu réactionnel est agité 48 h à température ambiante puis évaporé sous pression réduite. Le résidu est repris au chloroforme, lavé par NaOH 1N, la phase organique séchée sur $Na_2SO_4$ et évaporée pour donner une huile jaunâtre.

IR (C=O) : 1621 $cm^{-1}$ ($CHCl_3$).

c) cis 2-(4-méthyl pipéridinométhyl) cyclohexylamine.

La 1-[cis (2-aminocyclohexyl) carbonyl]-4-méthyl pipéridine (6,57 g ; 0,0293 mole) est dissoute dans du tétrahydrofuranne (25 ml) et ajoutée goutte à goutte à une suspension de $AlLiH_4$ (2,22 g ; 0,0586 mole) dans du tétrahydrofuranne (50 ml). En fin d'addition le milieu réactionnel est porté 24 h au reflux puis hydrolysé en additionnant avec précaution de l'eau (2,3 ml) puis une solution de soude à 15 % (2,3 ml) puis de l'eau (5ml).

Les sels d'aluminium sont éliminés par filtration et le tétrahydrofuranne évaporé sous vide. La résidu est repris par une solution d'acide rendue alcaline par une solution de soude à 15 %, extraite au chloroforme. La phase organique est séchée sur $Na_2SO_4$ et évaporée sous vide. Le résidu est purifié par distillation.

Eb (27 Pa) : 86-87° C.

EXEMPLE B

cis 2-(4,4-diméthyl pipéridinométhyl) cyclohexylamine.

a) 1-[cis 2-trifluoroacétamido cyclohexyl) carbonyl]-4,4-diméthyl pipéridine.

Le produit est obtenu suivant la méthode décrite à l'exemple A a) en utilisant la 4,4-diméthyl pipéridine. L'huile résiduelle est cristallisée dans un mélange éther de pétrole/acétate d'éthyle (80/20).

F : 97-106°C, IR (C=O) : 1717,8-1622,0 $cm_{-1}$ ($CHCl_3$).

b) 1-[cis (2-amino cyclohexyl) carbonyl]-4,4-diméthyl pipéridine.

Le produit obtenu en a) est détrifluoroacétylé suivant la méthode décrite à l'exemple A b) pour fournir une huile.

IR (C=O) : 1621 $cm^{-1}$ ($CHCl_3$).

c) cis 2-(4,4-diméthyl pipéridinométhyl) cyclohexylamine.

La 1-[cis (2-amino cyclohexyl) carbonyl]4,4-diméthyl pipéridine est réduite par AlLiH4 selon la procédure décrite à l'exemple A c). L'huile obtenue est distillée.

Eb (53 Pa) : 112° C.

Les autres dérivés cis de cyclohexylamine obtenus suivant les mêmes procédés et sont reportés dans le tableau II.

## TABLEAU II

| Exemples N° | Z | 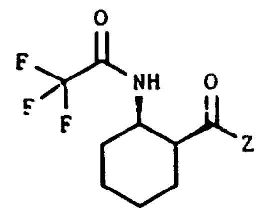 a) F*(°C) | IR $\nu$(C=O) $cm^{-1}$ | 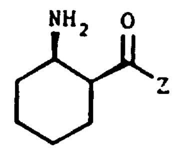 b) IR $\nu$(C=O) $cm^{-1}$ | 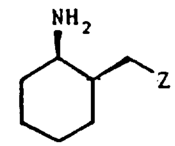 c) Eb** °C (Pa) |
|---|---|---|---|---|---|
| C | 1-pipéridinyle | 134-137 | 1717 ; 1620 ($CHCl_3$) | | |
| D | 1-(4-éthyl) pipéridinyle | 92-96 | 1719 ; 1621,5 ($CHCl_3$) | 1621,6 ($CHCl_3$) | 125 (27) |
| E | 1-(4-propyl) pipéridinyle | 113-114 | 1718 ; 1621,7 ($CHCl_3$) | 1621,7 ($CHCl_3$) | 125 (27) |
| F | 1-(4-t butyl) pipéridinyle | 151-152 | 1719 ; 1621,7 ($CHCl_3$) | 1622 ($CHCl_3$) | 150 (66) |
| G | 1-(4-éthyl-4-méthyl) pipéridinyle | 91-94 | 1718,3 ; 1621,7 ($CHCl_3$) | 1621,7 ($CHCl_3$) | 150 (53) |
| H | 1-(3,5-diméthyl)pipéridinyle | 118 | 1716 ; 1629 (KBr) | 1623 ($CHCl_3$) | |
| I | 1-(4-méthoxy-éthyl) pipéridinyle | 94,5 | 1718 ; 1622 ($CHCl_3$) | 1622 ($CHCl_3$) | 125 (53) |

* Banc de Köfler
** Four à boules Büchi

**Tableau II (suite)**

| Exemples N° | Z | 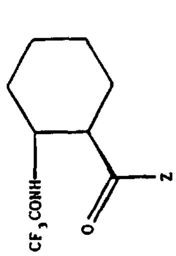 Fusion °C | IR δ $cm^{-1}$ | 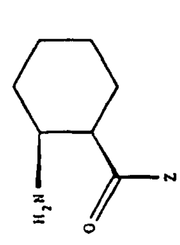 IR δ $cm^{-1}$ | 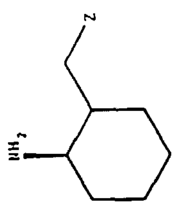 Eb |
|---|---|---|---|---|---|
| J | 1-(4-hydroxy) pipéridinyle | 135 | 1718-1625 ($CHCl_3$) | 1624 ($CHCl_3$) | huile ND |
| K | 1-(4-hydroxy éthyl) pipéridinyle | huile | 1717-1623 ($CHCl_3$) | 1622 ($CHCl_3$) | huile ND |
| L | 1-(4-éthoxy)-pipéridinyle | 94-96 | 1719-1624 ($CHCl_3$) | 1625 ($CHCl_3$) | 137,5°C (0,4 mmHg) |
| M | 1-(4-méthoxy méthyl)-pipéridinyle | 132-133 ($CHCl_3$) | 1718-1624 | 1624 ($CHCl_3$) | huile ND |

ND : Non Distillée

EXEMPLE N

Trans 2-(4-méthylpipéridinométhyl) cyclohexylamine.

a) 2-(4-méthylpipéridinométhyl) cyclohexylamine.

La cyclohexanone (47,5 g ; 0,484 mole) est dissoute dans de l'éthanol à 95 % contenant du formaldéhyde (36 g d'une solution aqueuse à 37 %), de l'acide chlorhydrique (1,7 ml d'une solution aqueuse à 36 %) et de la 4-méthyl pipéridine (40 g ; 0,4 mole). La solution est portée au reflux quatre heures. L'éthanol est évaporé sous vide et le résidu repris par une solution d'acide chlorhydrique 2N. La phase aqueuse est lavée à l'éther, alcalinisée par une solution aqueuse de soude à 15 %, puis extraite par du chlorure de méthylène. La phase organique est séchée sur $Na_2SO_4$, puis le solvant chassé sous vide.

L'huile obtenue est distillée.

Eb (27 Pa) : 85-90° C
IR (C=O) : 1706 ($CHCl_3$).

b) 2-(4-méthylpipéridinométhyl) cyclohexanone oxime.

La 2-(4-méthylpipéridinométhyl) cyclohexanone (20,8 g ; 0,1 mole) est dissoute dans l'éthanol (100 ml). A cette solution est ajouté le chlorhydrate d'hydroxylamine (7,54 g : 0,109 mole) en solution dans l'eau (15 ml). Le milieu réactionnel est agité 1 h à température ambiante. Le résidu est repris par le chloroforme et lavé par une solution aqueuse de soude 2N. La phase organique est séchée sur $Na_2SO_4$ et évaporée pour fournir une poudre blanche.

F : 113-116,5° C.

c) 2-(4-méthylpipéridinométhyl) cyclohexylamine.

L'oxime (13,5 g ; 0,06 mole) obtenu à l'exemple précédent est dissous dans l'alcool amylique (200 ml). Du sodium (8,33 g ; 0,36 mole) est ajouté par petite quantité de façon à maintenir une ébullition douce. En fin d'addition, le milieu réactionnel est refroidi puis acidifié par une solution aqueuse d'HCl 2N. La phase aqueuse est extraite par l'acétate d'éthyle, alcalinisée par une solution de soude à 15 %, puis extraite au chlorure de méthylène. La phase organique est séchée sur $Na_2SO_4$ puis évaporée. L'huile résiduelle est distillée.
Eb (27 Pa) : 94-98° C. Le produit obtenu contient environ 75 % de trans 2-(4-méthylpipéridinométhyl) cyclohexylamine. Le diastéréoisomère pur est isolé au niveau du benzamide.

## EXEMPLE O

(S,S) et (R,R)2-(4 méthyl pipéridinométhyl) cyclohexylamine.

a) 1-[2(R)-benzamido-(S) cyclohexyl carbonyl]-4-méthyl pipéridine.

L'acide 2-(R) benzamido-(S) cyclohexylcarboxylique (8,87 g) est dissous dans 200 ml de tétrahydrofuranne. Après addition de carbonyldiimidazole (5,82 g), le milieu réactionnel est agité 1 h 30 à température ambiante. Une solution de 4-méthyl pipéridine dans 20 ml de tétrahydrofuranne est ajoutée et le milieu réactionnel encore agité 48 heures. Le solvant est chassé sous vide, le résidu est repris dans le chlorure de méthylène. La phase organique est lavée par 2 fois 50 ml d'HCl 1N, 2 fois 50 ml de NaOH 1N, puis 2 fois 50 ml d'eau, séchée sur $Na_2SO_4$ et évaporée sous pression réduite. On obtient 11,16 g d'huile (94 %) $[\alpha]_{578}^{20}$ = -47,9° (c = 1, meOH); IR: $\nu$ = 1731 faible, 1648, 1620 $cm^{-1}$ ($CHCl_3$).

a') A partir de l'acide antipode, par le même procédé, on obtient le 1-[2(S)-benzamido-(R) cyclohexylcarbonyl]-4-méthyl pipéridine.
$[\alpha]_{578}^{20}$ = +49,2° ; (c = 1, MeOH) ; IR $\nu$ = 1730 (faible), 1648, 1619 $cm^{-1}$ ($CHCl)_3$.

b) N-benzoyl-2(R)-(4-méthyl pipéridino méthyl) (R) cyclohexylamine.

Le produit obtenu à l'étape a) (10,38 g) en solution dans 20 ml de tétrahydrofuranne est additionné goutte à goutte à une suspension de $AlLiH_4$ (2,4 g) dans 130 ml de tétrahydrofuranne refroidie à 0° C. En fin d'addition le mélange est porté 45 minutes à 60° C. Le milieu est refroidi, puis hydrolysé par une solution aqueuse de NaOH 1N (12 ml). L'insoluble est éliminé par filtration et le filtrat évaporé sous vide. Le résidu est dissous dans l'acide chlorhydrique 1N. La phase aqueuse est lavée par de l'acétate d'éthyle, puis alcalinisée par la soude 2N. Le produit est extrait par l'acétate d'éthyle. La phase organique est séchée sur $Na_2SO_4$, puis évaporée sous pression réduite. Le produit recherché est cristallisé dans un mélange éther de pétrole - acétate d'éthyle (95-5), et collecté par filtration = 6,53 g (65 %).

$[\alpha]_{578}^{20}$ = -67,1° (c = 1, MeOH).
PF = 89-91° C.

b') A partir du produit obtenu en a'), par le même procédé, on obtient le N-benzoyl-2(S)-(4-méthyl pipéridinométhyl)-(S) cyclohexylamine.

$[\alpha]_{578}^{20}$ = +67,2°
PF = 79,5-84,5° C.

c) (R,R)2-(4-méthyl pipéridinométhyl) cyclohexylamine.

Le produit obtenu à l'étape b) (6,43 g) est dissous dans 55 ml d'HCl 6N. Le mélange est porté au reflux pendant 5 jours. Le milieu est extrait par l'acétate d'éthyle, alcalinisé par une solution d'hydroxyde de sodium concentrée et extraite par trois fois 100 ml d'acétate d'éthyle. La phase organique est séchée sur $Na_2SO_4$, puis évaporée sous pression réduite. La diamine est obtenue sous forme d'huile (4,17 g ; 97 %).
$[a]_D^{20}$ = -25,2° (c = 1, MeOH).

c') A partir du produit obtenu en b'), le même procédé fournit le (S,S) 2-(4-méthyl pipéridinométhyl) cyclohexylamine.
$[a]_D^{20}$ = +25,2 (c = 1, MeOH).

On donnera ci-après des résultats mettant en évidence les propriétés des composés de formule I dans deux tests, à savoir vidange gastrique des solides et antagonisme des stéréotypies induites par l'apomorphine. Le premier test permet de mettre en évidence l'activité des produits sur la motricité digestive, le second permet de vérifier l'absence d'effets antagonistes dopaminergiques centraux.

Activité sur la motricité digestive

La vidange gastrique est étudiée chez des rats Sprague Dawley mâles de 150 g environ. A JO, une vagotomie post-diaphragmatique est pratiquée de façon à créer un modèle de vidange gastrique ralentie. A J7, les animaux reçoivent par voie oesophagienne ou intrapéritonéale le produit à tester ou le placebo (TO). A T + 15 minutes, n particules indigestibles d'1 mm de diamètre sont administrées par voie intra- gastrique. Les animaux sont euthanasiés à T + 3 h et les particules restant dans l'estomac sont dénombrées. La vidange gastrique est exprimée par le pourcentage de particules ayant quitté l'estomac.

Elle est inférieure à 30 % chez les rats ayant subi une vagotomie ; par comparaison, elle est supérieure à 50 % chez les animaux n'ayant subi qu'une laparotomie.

Pour chaque composé, 3 doses au moins sont testées (10 animaux minimum par dose), un témoin positif (métoclopramide 5 mg $kg^{-1}$) est toujours réalisé en parallèle. Les comparaisons statistiques font appel au test non paramétrique de Mann et Whitney.

Les résultats obtenus sont présentés au tableau III. Lorsqu'un produit s'avère actif, on détermine sa dose minimale efficace (DME) et la potentialisation (sous forme d'un multiple de la valeur témoin) observée à cette dose. Si le produit est actif dès la plus faible dose testée, on précise que la DME est inférieure ou égale ($\leq$) à cette dose.

Activité antagoniste dopaminergique centrale

Des stéréotypies de la sphère buccale sont induites chez des rats Sprague Dawley mâles de 150 g environ, par l'injection sous-cutanée de chlorhydrate d'apomorphine (1 mg $kg^{-1}$). Les produits à étudier sont administrés par voie intra-péritonéale 30 minutes avant l'injection d'apomorphine. Les animaux sont observés 15, 30, 45, 60, 75 et 90 minutes après cette injection.

L'intensité des stéréotypies est évaluée par un système de cotation allant de 0 (absence de mouvements anormaux) à 3 (léchage et/ou mâchonnements intenses ou permanents) et exprimée sous forme d'un score cumulé en 90 minutes.

Pour chaque produit, 2 doses au moins sont testées (6 animaux minimum par dose), un témoin positif (métoclopramide 3 mg $kg^{-1}$) est toujours réalisé en parallèle. Les comparaisons statistiques font appel au test non paramétrique de Mann et Whitney. Les résultats obtenus sont présentés au tableau III. Lorsque les produits sont inactifs (IN), on précise la plus forte dose testée ; lorsqu'un produit s'avère actif, on précise la dose efficace (D.E.) et le pourcentage d'inhibition observé.

TABLEAU III

| Exemples | Vidange Gastrique des Solides | | Antagonisme des Stéréotypies induites par l'Apomorphine D.E. (mg $kg^{-1}$) |
|---|---|---|---|
| | D.M.E. ($\mu g\ kg^{-1}$) | Potentialisation | |
| 2 | ≤ 10 | x 2,5 | IN (3) |
| 3 | 30 | x 2,5 | IN (3) |
| 4 | ≤ 10 | x 3,4 | IN (3) |
| 5 | ≤ 30 * | x 3,1 | IN (3) |
| 6 | 300 * | x 1,9 | IN (3) |
| 7 | ≤ 30 | x 3,9 | IN (3) |
| 8 | ≤ 30 | x 2,3 | IN (3) |
| 9 | ≤ 10 | x 3,3 | IN (3) |
| 10 | ≤ 10 | x 6,1 | IN (3) |
| 11 | ≤ 10 | x 5,4 | IN (3) |
| 12 | 30 | x 2 | IN (3) |
| 13 | ≤ 30 | x 4,2 | IN (3) |
| 14 | 1000 * | x 2,6 | IN (3) |
| 15 | 30 | x 5,7 | IN (3) |
| 16 | 3000 * | x 3,2 | IN (3) |
| Composé A GB-A-1 015 921 | 3000 | NS** | |

* produit étudié après administration intra-péritonéale

** non significatif

Tableau III (suite)

| Exemples | VIDANGE GASTRIQUE DES SOLIDES | | Antagonisme des stéréotypies induites par l'apomorphine DE ($mg.k^{-1}$) |
|---|---|---|---|
| | DME ($\mu g.kg^{-1}$) | Potentialisation | |
| 17 | 30 | 1,5 | IN (3) |
| 18 | 10 | 2,5 | IN (10) |
| 19 | 10 | 2,8 | ND |
| 20 | 10 | 3,1 | ND |
| 21 | 10 | 1,9 | ND |
| 22 | 30 | 1,6 | ND |
| 23 | < 10 | 1,7 | IN (3) |
| 24 | 30 | 2 | IN (3) |
| 25 | 30 | 1,7 | IN (3) |
| 27 | 10 | 2,1 | ND |
| 28 | < 10 | 2,1 | IN (10) |
| 29 | 30 | 2,7 | ND |
| 30 | 10 | 1,9 | IN (3) |
| 31 | 30 | 2,7 | IN (3) |
| 32 | 50 | x 7 | IN (1) |
| 33 | 30 | 2,6 | ND |

ND : Non Déterminé

Les composés de formule (I) peuvent être utilisés pour stimuler la motricité gastro-intestinale. Ils peuvent être utilisés pour traiter les maladies de la sphère digestive telles que la gastroparésie, le reflux gastro-oesophagien, les dyspepsies, la constipation et certaines formes du syndrôme du côlon ou de l'intestin irritable.

On peut administrer chez l'homme ces composés ou leurs sels d'addition obtenus avec des acides pharmaceutiquement acceptables sous une grande variété de compositions pharmaceutiques.

La présente invention a donc également pour objet des compositions thérapeutiques comprenant à titre de principe actif un composé de formule I ou l'un de ses sels d'addition avec des acides pharmaceutiquement acceptables.

Le principe actif peut être administré par voie orale, intraveineuse, sous-cutanée ou parentérale en une ou plusieurs fois pour un dosage journalier de 0,05 mg à 50 mg.

Les compositions thérapeutiques peuvent contenir 0,05 à 50 mg de principe actif par unité. Pour l'administration

par voie orale, elles peuvent être présentées sous forme de tablettes ou de gélules contenant un des vecteurs couramment employés dans l'art pharmaceutique.

## Revendications

**1.** Composés de formule

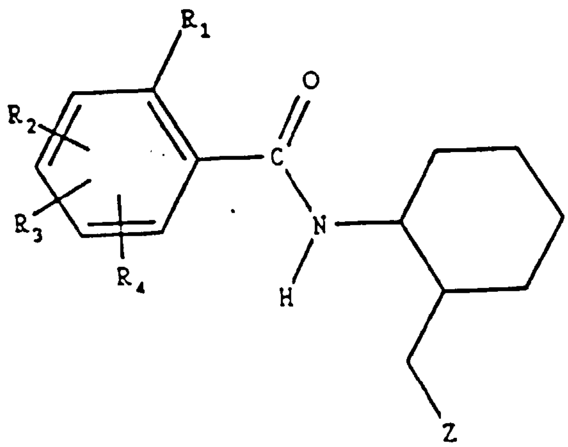

(I)

dans laquelle :

$R_1$ est choisi parmi les groupes alkoxy en $C_1$-$C_4$, méthoxy alkoxy en $C_1$-$C_3$, alkenyloxy en $C_3$-$C_4$ et cyclo alkyloxy en $C_5$-$C_6$,
ou $R_1$ et $R_2$, en position 3, forment ensemble et avec le noyau aromatique sur lequel ils sont fixés un cycle 2,3-dihydrobenzofuranne,
$R_2$, $R_3$, $R_4$ sont choisis indépendamment l'un de l'autre parmi un atome d'hydrogène, un atome d'halogène et les groupes hydroxy, alkoxy en $C_1$-$C_3$, amino, (alkyl en $C_1$-$C_3$) amino, di(alkyl en $C_1$-$C_3$) amino et (alkyl en $C_1$-$C_3$) carbonyl amino,
Z est choisi parmi les groupes pipéridinyle et pipéridinyle substitué en 4 par un groupe hydroxy, alkoxy en $C_1$-$C_3$, hydroxyéthyle, (alkoxy en $C_1$-$C_3$)éthyle, (alkoxy en $C_1$-$C_3$) méthyle, un groupe alkyle en $C_1$-$C_4$ ou deux groupes alkyle en $C_1$-$C_3$,
leurs N-oxydes,

et leurs sels d'addition avec des acides pharmaceutiquement acceptables.

**2.** Composés selon la revendication 1, dans laquelle :

$R_1$ est choisi parmi les groupes alkoxy en $C_1$-$C_4$, méthoxy alkoxy en $C_1$-$C_3$, alkenyloxy en $C_3$-$C_4$ et cyclo alkyloxy en $C_5$-$C_6$,
ou $R_1$ et $R_2$, en position 3, forment ensemble et avec le noyau aromatique sur lequel ils sont fixés un cycle 2,3-dihydrobenzofuranne,
$R_2$, $R_3$, $R_4$ sont choisis indépendamment l'un de l'autre parmi un atome d'hydrogène, un atome d'halogène et les groupes hydroxy, alkoxy en $C_1$-$C_3$, amino, (alkyl en $C_1$-$C_3$) amino, di(alkyl en $C_1$-$C_3$) amino et (alkyl en $C_1$-$C_3$) carbonyl amino,
Z est choisi parmi les groupes pipéridinyle et pipéridinyle substitué en 4 par un groupe alkoxy en $C_1$-$C_3$, (alkoxy en $C_1$-$C_3$) éthyle, (alkoxy en $C_1$-$C_3$) méthyle, un groupe alkyle en $C_1$-$C_4$ linéaire ou ramifié, ou deux groupes alkyle en $C_1$-$C_3$,

leurs N-oxydes,
et leurs sels d'addition avec des acides pharmaceutiquement acceptables.

**3.** Composés selon la revendication 1, de formule

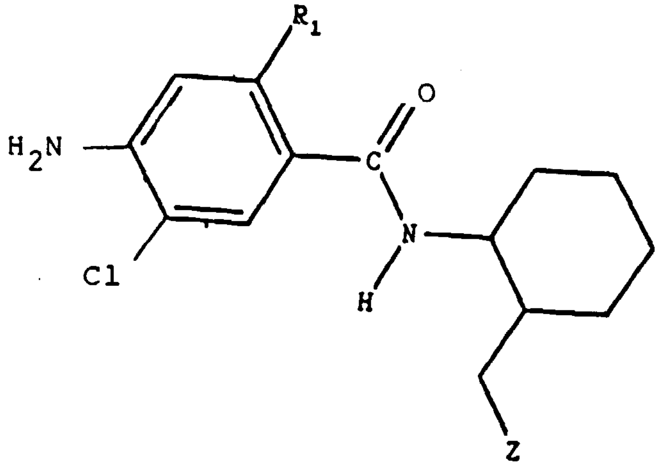

dans laquelle $R_1$ est un groupe alkoxy en $C_1$-$C_4$.

**4.** Composés selon la revendication 3 dans laquelle $R_1$ est un groupe méthoxy.

**5.** Composés selon la revendication 3, dans laquelle $R_1$ est un groupe éthoxy ou cyclopropylméthoxy.

**6.** Composés selon l'une quelconque des revendications 3, 4 ou 5, dans laquelle Z est un groupe pipéridino, 4-méthylpipéridino, 4,4-diméthylpipéridino ou 4-hydroxypipéridino.

**7.** Composés selon la revendication 6, de formule

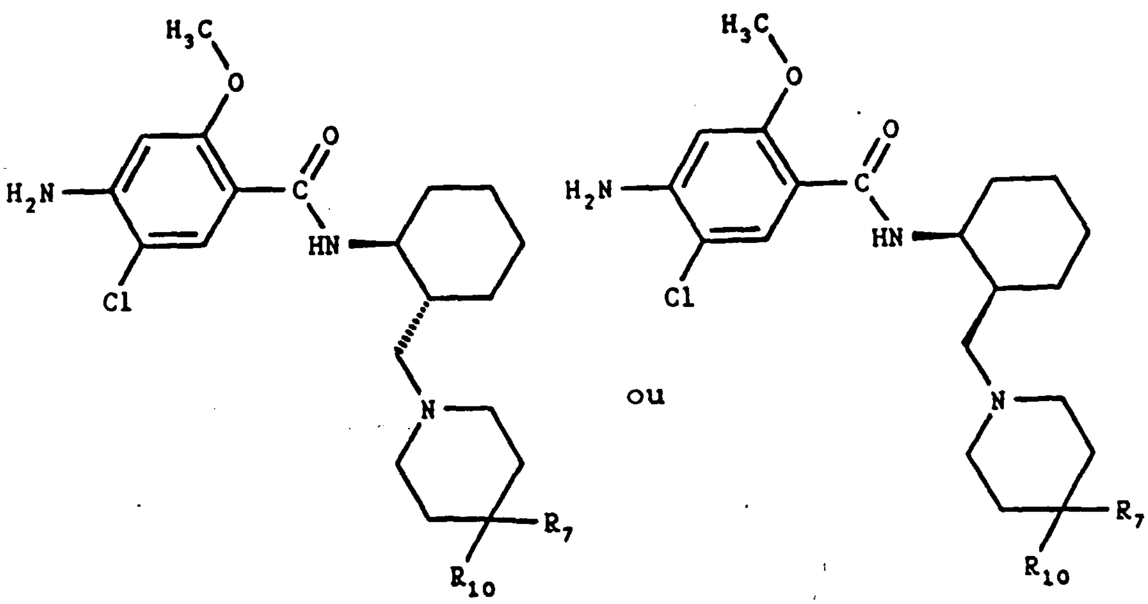

ou

(IV) (V)

dans laquelle $R_7$ est un groupe méthyle et $R_{10}$ est un atome d'hydrogène.

**8.** Composés selon la revendication 6, de formule

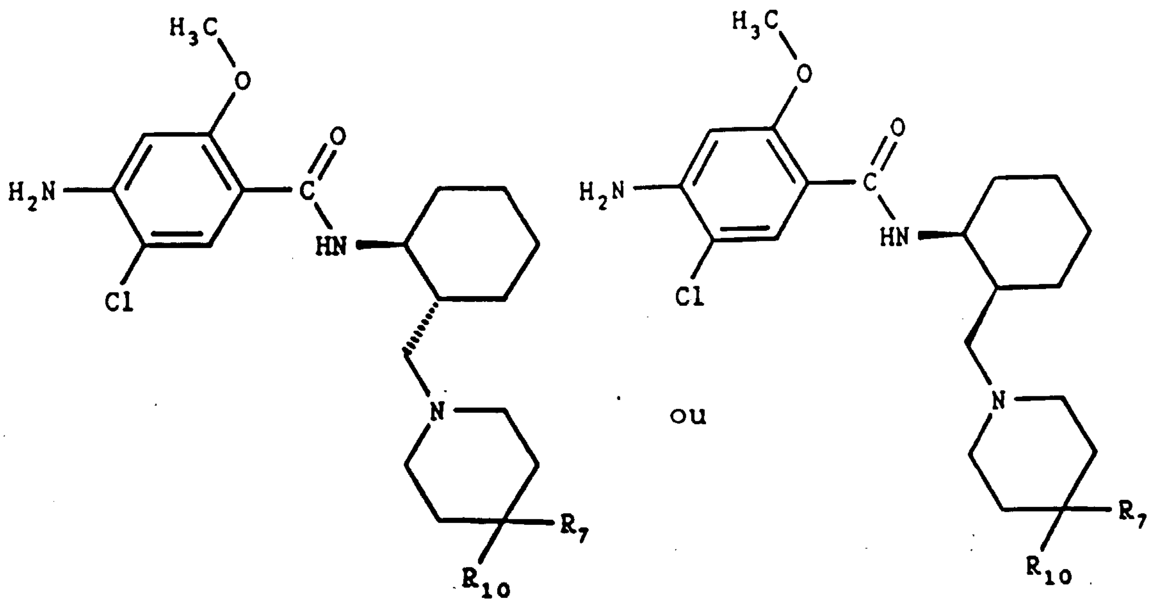

ou

(IV) (V)

dans laquelle $R_7$ et $R_{10}$ sont des groupes méthyle.

**9.** Composés selon la revendication 6, de formule

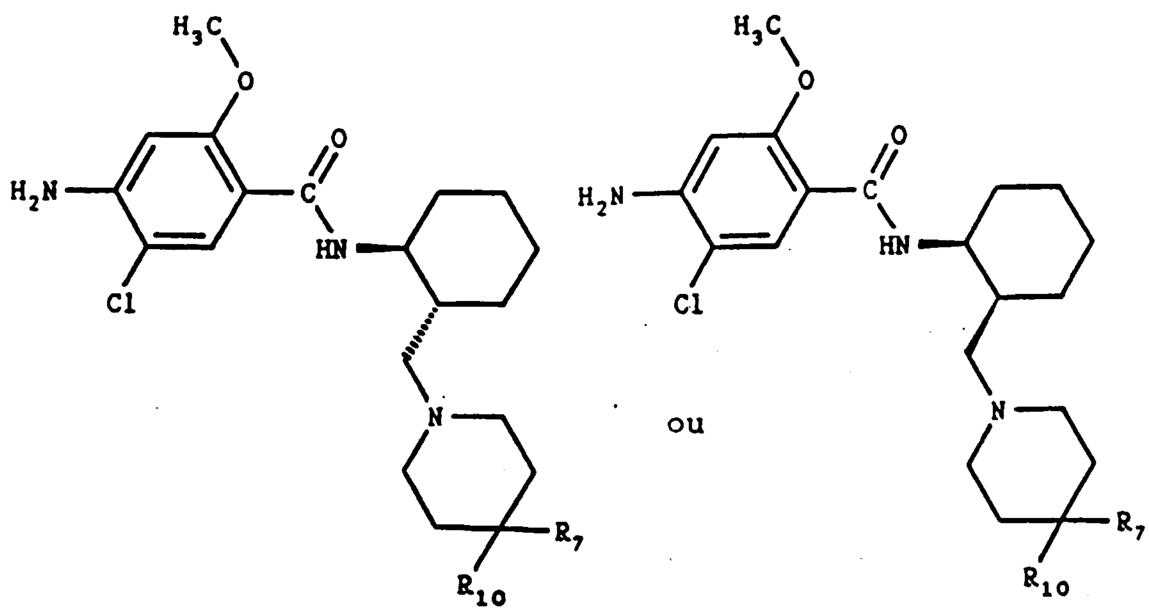

ou

(IV) (V)

dans laquelle $R_7$ est un groupe hydroxy et $R_{10}$ un atome d'hydrogène.

**10.** Procédé de préparation d'un composé de formule I selon la revendication 1, dans lequel on effectue une condensation entre un acide benzoïque de formule

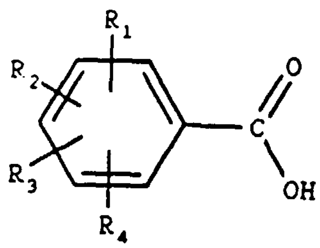

(VI)

dans laquelle $R_1$, $R_2$, $R_3$ et $R_4$ ont la signification donnée à la revendication 1, avec une cyclohexylamine de formule

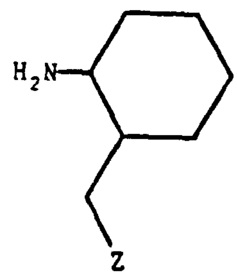

(VII)

dans laquelle Z a la signification donnée à la revendication 1.

**11.** Procédé selon la revendication 10, dans lequel on effectue la réaction en présence d'un agent de couplage choisi parmi le carbonyldiimidazole et le dicyclohexylcarbodiimide.

**12.** Composition thérapeutique comprenant à titre de principe actif un composé selon l'une quelconque des revendications 1 à 9.

**Patentansprüche**

**1.** Verbindungen der Formel

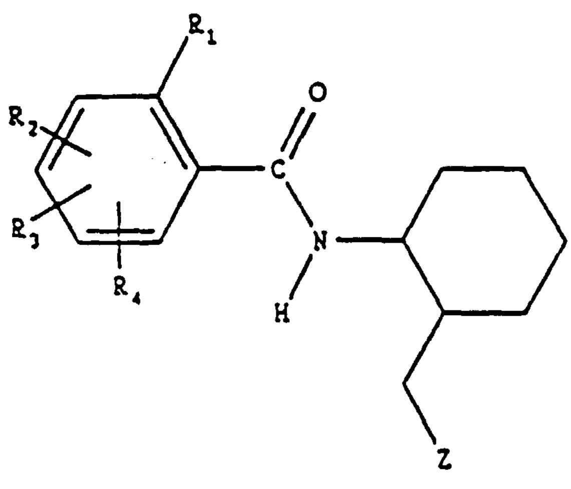

(I)

in der:

$R_1$ aus ($C_1$-$C_4$) -Alkoxy-, Methoxy-($C_1$-$C_3$) -alkoxy-, ($C_3$-$C_4$)-Alkenyloxy- und ($C_5$-$C_6$)-Cycloalkyloxygruppen ausgewählt ist oder $R_1$ und $R_2$ in der Position 3 zusammen mit dem aromatischen Ring, an dem sie angebracht sind, einen 2,3-Dihydrobenzofuranzyklus bilden,
$R_2$, $R_3$, $R_4$ unabhängig voneinander ausgewählt sind aus einem Wasserstoffatom, einem Halogenatom und Hydroxy-, ($C_1$-$C_3$)-Alkoxy-, Amino-, ($C_1$-$C_3$) -Alkylamino-, Di-($C_1$-$C_3$)-alkylamino- und ($C_1$-$C_3$)-Alkylcarbonylaminogruppen,
Z ausgewählt ist aus der Piperidinylgruppe und einer Piperidinylgruppe, die in 4 mit einer Hydroxy-, ($C_1$-$C_3$)-Alkoxy-, Hydroxyethyl-, ($C_1$-$C_3$) -Alkoxyethyl-, ($C_1$-$C_3$)-Alkoxymethylgruppe, einer ($C_1$-$C_4$)-Alkylgruppe oder zwei ($C_1$-$C_3$)-Alkylgruppen substituiert ist,

ihre N-Oxide
und ihre Additionssalze mit pharmazeutisch annehmbaren Säuren.

**2.** Verbindungen nach Anspruch 1, worin:

$R_1$ ausgewählt ist aus ($C_1$-$C_4$)-Alkoxy-, Methoxy-($C_1$-$C_3$)-alkoxy-, ($C_3$-$C_4$)-Alkenyloxy- und ($C_5$-$C_6$)-Cycloalkyloxygruppen
oder $R_1$ und $R_2$ in Position 3 zusammen mit dem aromatischen Ring, an dem sie angebracht sind, einen 2,3-Dihydrobenzofuranzyklus bilden,
$R_2$, $R_3$, $R_4$ unabhängig voneinander ausgewählt sind aus einem Wasserstoffatom, einem Halogenatom und Hydroxy-, ($C_1$-$C_3$)-Alkoxy-, Amino-, ($C_1$-$C_3$)-Alkylamino-, Di-($C_1$-$C_3$)-alkylamino- und ($C_1$-$C_3$)-Alkylcarbonylaminogruppen,
Z ausgewählt ist aus der Piperidinylgruppe und einer Piperidinylgruppe, die in 4 durch eine ($C_1$-$C_3$)-Alkoxy-, ($C_1$-$C_3$) -Alkoxyethyl-, ($C_1$-$C_3$)-Alkoxymethylgruppe, eine lineare oder verzweigte ($C_1$-$C_4$)-Alkylgruppe oder zwei ($C_1$-$C_3$)-Alkylgruppen substituiert ist,

ihre N-Oxide
und ihre Additionssalze mit pharmazeutisch annehmbaren Säuren.

**3.** Verbindungen nach Anspruch 1 der Formel

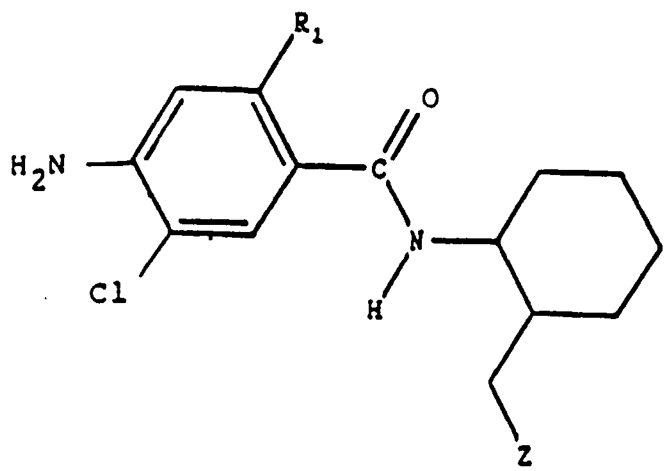

in der $R_1$ eine ($C_1$-$C_4$)-Alkoxygruppe ist.

**4.** Verbindungen nach Anspruch 3, worin $R_1$ eine Methoxygruppe ist.

**5.** Verbindungen nach Anspruch 3, worin $R_1$ eine Ethoxy- oder Cyclopropylmethoxygruppe ist.

**6.** Verbindungen nach irgendeinem der Ansprüche 3, 4 oder 5, worin Z eine Piperidino-, 4-Methylpiperidino-, 4,4-Dimethylpiperidino- oder 4-Hydroxypiperidinogruppe ist.

**7.** Verbindungen nach Anspruch 6 der Formel

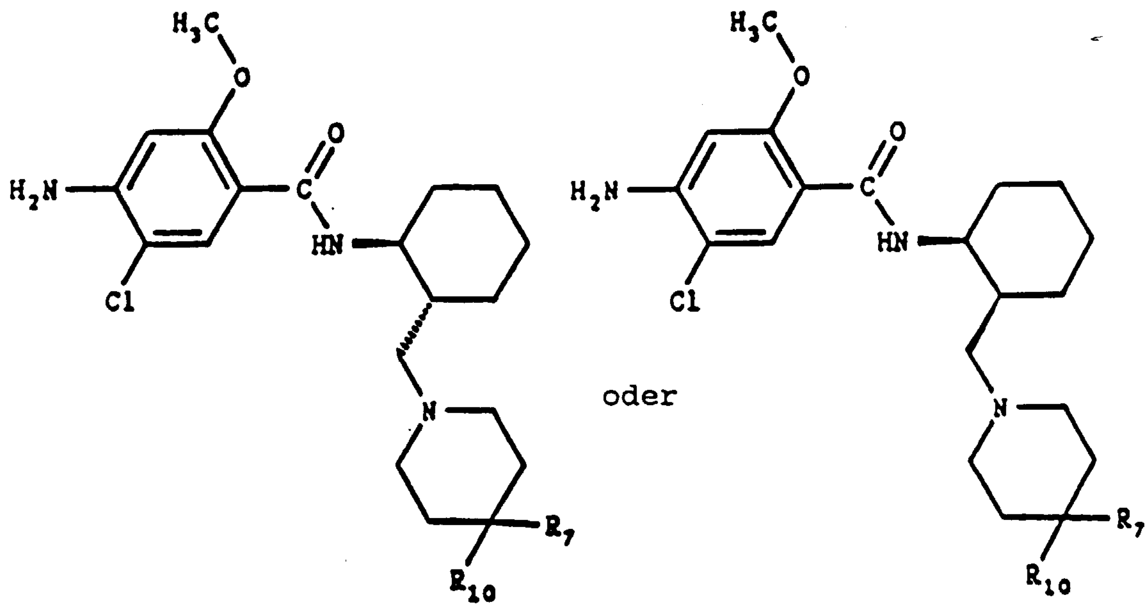

oder

(IV) (V)

in der $R_7$ eine Methylgruppe ist und $R_{10}$ ein Wasserstoffatom ist.

**8.** Verbindungen nach Anspruch 6 der Formel

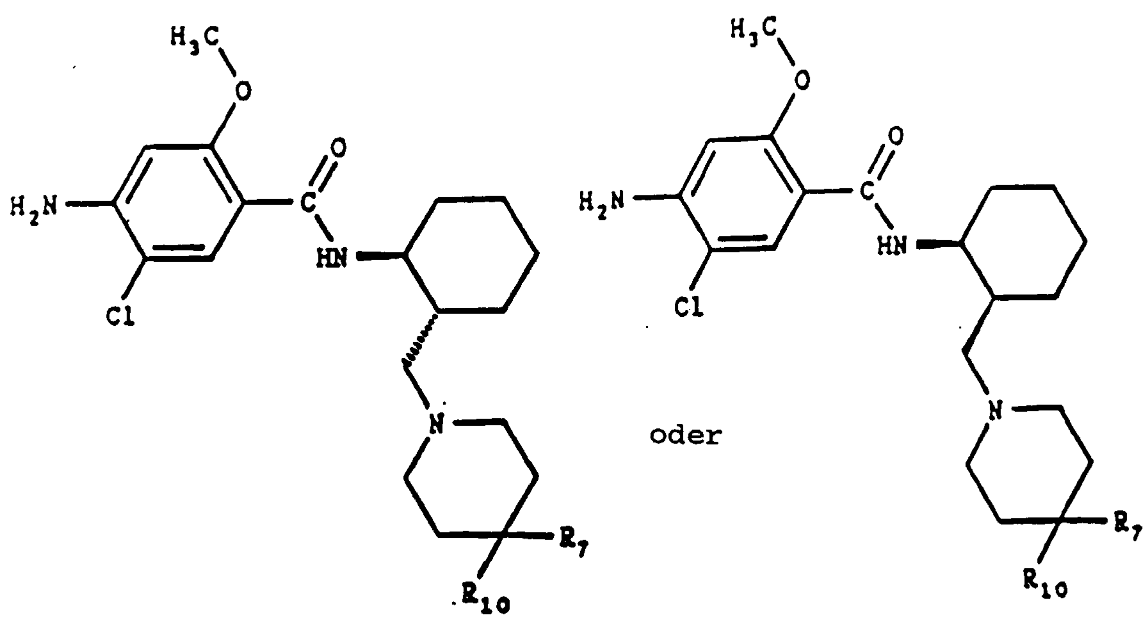

oder

(IV) (V)

in der $R_7$ und $R_{10}$ Methylgruppen sind.

**9.** Verbindungen nach Anspruch 6 der Formel

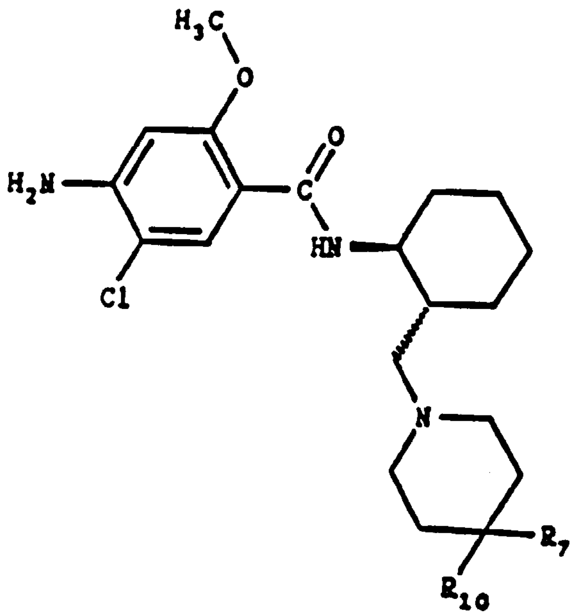

oder

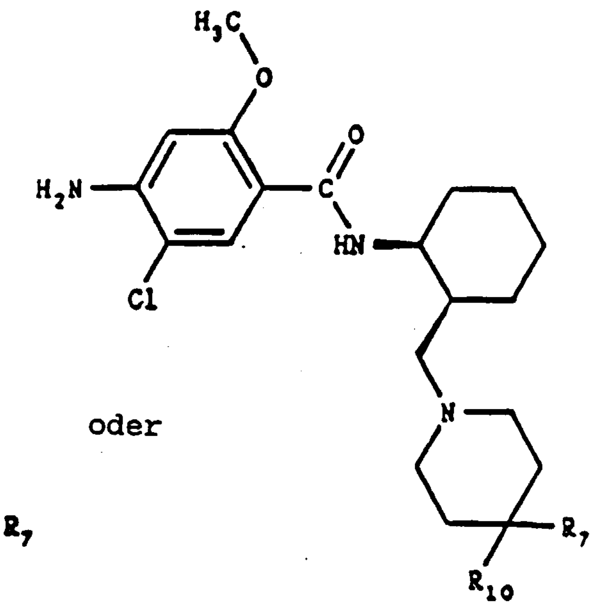

(IV) (V)

in der $R_7$ eine Hydroxygruppe ist und $R_{10}$ ein Wasserstoffatom ist.

**10.** Verfahren zur Herstellung einer Verbindung der Formel I nach Anspruch 1, in dem man eine Kondensation zwischen einer Benzoesäure der Formel

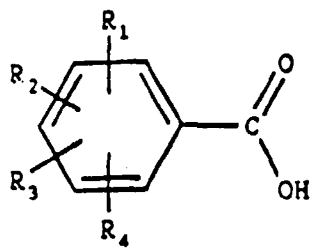

(VI)

in der $R_1$, $R_2$, $R_3$ und $R_4$ die in Anspruch 1 angegebene Bedeutung aufweisen,
mit einem Cyclohexylamin der Formel

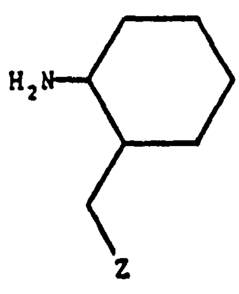

(VII)

in der Z die in Anspruch 1 angegebene Bedeutung aufweist, bewirkt.

**11.** Verfahren nach Anspruch 10, in dem man die Reaktion in Anwesenheit eines Kupplungsmittels bewirkt, das aus Carbonyldiimidazol und Dicyclohexylcarbodiimid ausgewählt ist.

**12.** Therapeutische Zusammensetzung, die als Wirkstoff eine Verbindung nach irgendeinem der Ansprüche 1 bis 9 umfaßt.

## Claims

**1.** Compounds of the formula

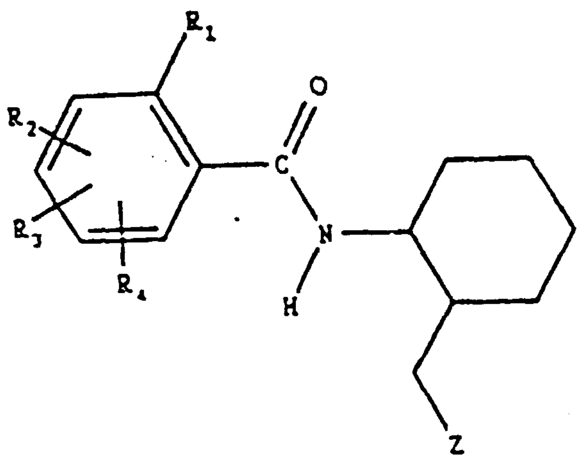

(I)

in which:

$R_1$ is selected from among the alkoxy $C_1$ - $C_4$, methoxy alkoxy $C_1$ - $C_3$, and cyclo alkyloxy $C_5$ - $C_6$ groups, wherein $R_1$ and $R_2$ in position 3 form, together and with the aromatic core upon which they are fixed, a 2,3-dihydrobenzofuranne cycle,
$R_2$, $R_3$, $R_4$ are selected independently of one another from among a hydrogen atom, a halogen atom and the hydroxy, alkoxy $C_1$ - $C_3$, amino, (alkyl $C_1$ - $C_3$) amino, di(alkyl $C_1$-$C_3$) amino, and (alkyl $C_1$ - $C_3$) carbonyl amino groups,
Z is selected from among the groups piperidinyl and piperidinyl substituted at 4 by a hydroxy, alkoxy $C_1$ - $C_3$, hydroxyethyl, (alkoxy $C_1$ - $C_3$) ethyl, (alkoxy $C_1$ - $C_3$) methyl, an alkyl $C_1$ - $C_4$ group or two alkyl $C_1$ - $C_3$ groups,

their N-oxides,
and their salts of addition with pharmaceutically acceptable acids.

**2.** Compounds according to claim 1, in which:

$R_1$ is selected from among the alkoxy $C_1$ - $C_4$ , methoxy alkoxy $C_1$ - $C_3$, alkenyloxy $C_3$ - $C_4$ and cyclo alkyloxy $C_5$ - $C_6$ groups,
wherein $R_2$ and $R_2$, in position 3, form together and with the aromatic core upon which they are fixed, a 2,3-dihydrobenzofuranne cycle,
$R_2$, $R_3$, $R_4$ are selected independently of one another from among a hydrogen atom, a halogen atom and the hydroxy, alkoxy $C_1$ - $C_3$, amino, (alkyl $C_1$ - $C_3$) amino, di(alkyl $C_1$-$C_3$) amino, and (alkyl $C_1$ - $C_3$) carbyl amino groups,
Z is selected from among the groups piperidinyl and piperidinyl substituted at 4 by an alkoxy $C_1$ - $C_3$, (alkoxy $C_1$ - $C_3$) ethyl, (alkoxy $C_1$ - $C_3$) methyl, a linear or branched alkyl $C_1$ - $C_4$ group, or two alkyl $C_1$ - $C_3$ groups,

their N-oxides,
and their salts of addition with pharmaceutically acceptable acids.

**3.** Compounds according to claim 1, of the formula

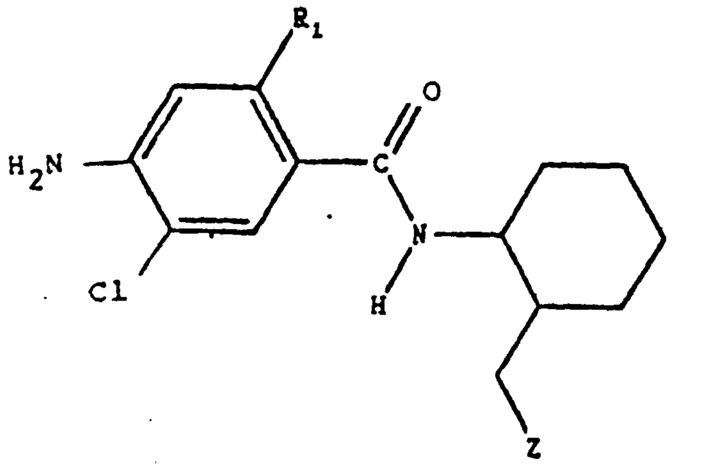

in which $R_1$ is an alkoxy $C_1$ - $C_4$ group.

**4.** Compounds according to claim 3 in which $R_1$ is a methoxy group.

**5.** Compounds according to claim 4 in which $R_1$ is an ethoxy or cyclopropylmethoxy group.

**6.** Compounds according to any one of claims 3, 4 or 5, in which Z is a piperidino, 4-methylpiperidino, 4,4-dimethylpiperidino or 4-hydroxypiperidino group.

**7.** Compounds according to claim 6, of the formula

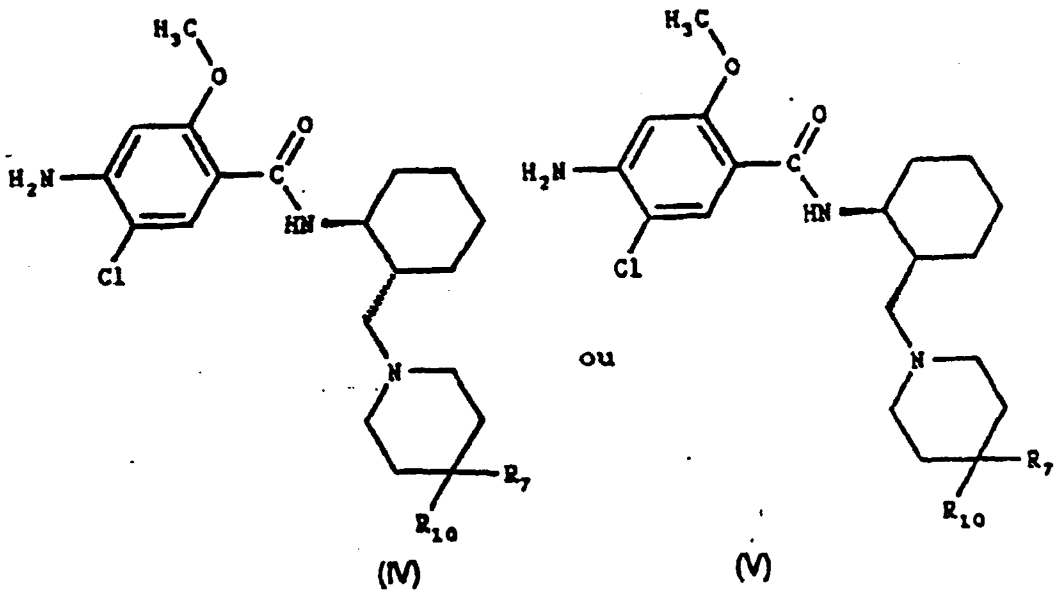

ou

(IV) (V)

in which $R_1$ is a methyl group and $R_{10}$ is a hydrogen atom.

**8.** Compounds according to claim 6, of the formula

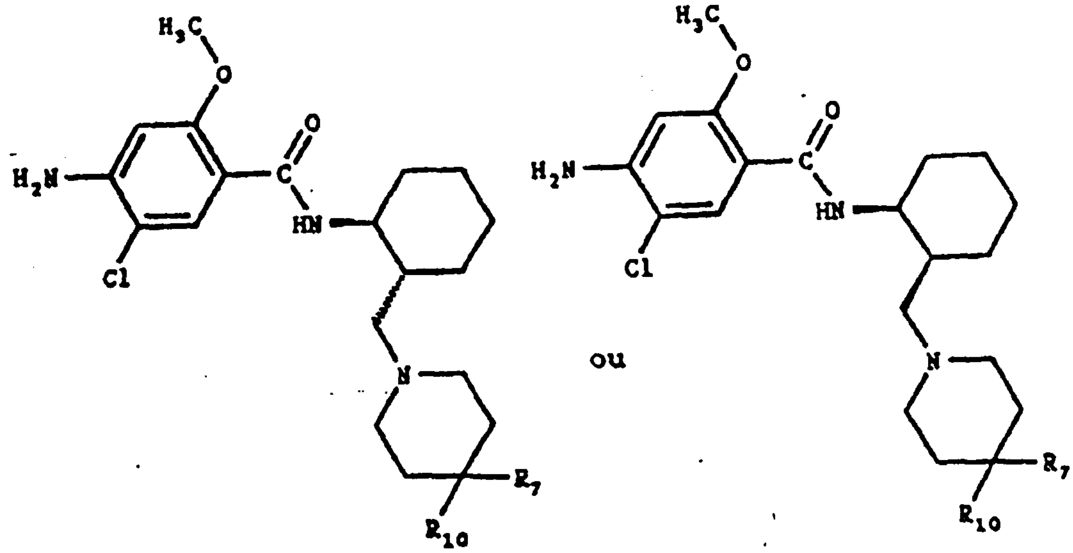

ou

(IV) (V)

in which $R_7$ and $R_{10}$ are methyl groups.

**9.** Compounds according to claim 6, of the formula

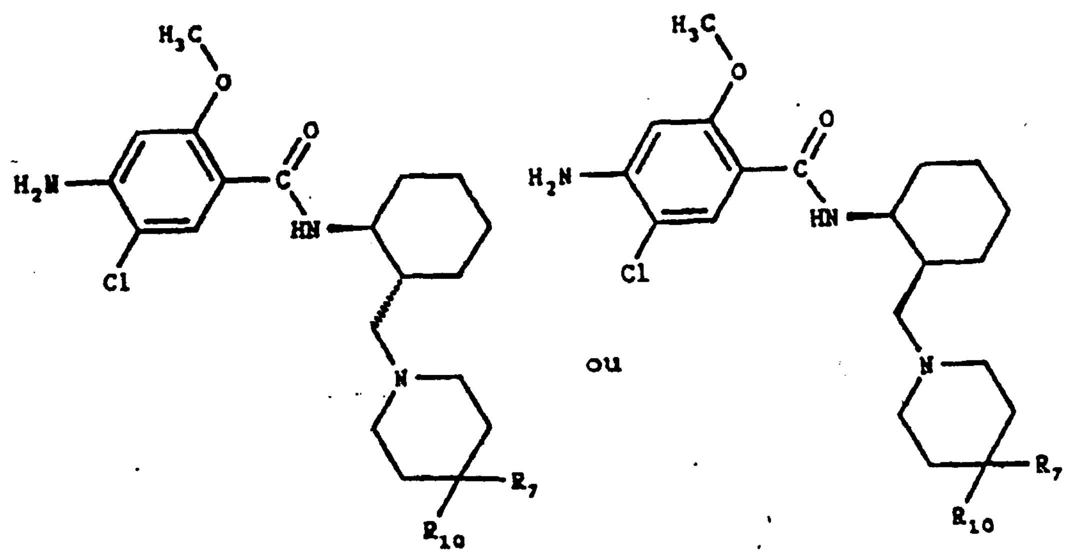

ou

(IV) (V)

in which $R_7$ is a hydroxy group and $R_{10}$ is a hydrogen atom.

**10.** Method for preparation of a compound of forumula I according to claim 1, in which condensation is effected between a benzoic acid of the formula

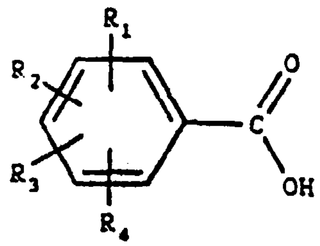

(VI)

in which $R_1$, $R_2$, $R_3$ and $R_4$ have the meaning given in claim 1, with a cyclohexamine of the formula

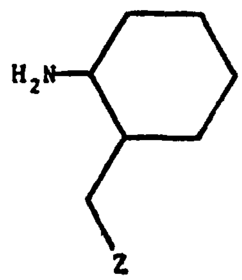

(VII)

in which Z has the meaning given in claim 1.

**11.** Method according to claim 10, in which the reaction is carried out in the presence of a coupling agent seleced from among carbonyldiimidazole and dicyclohexylcarbodiimide.

**12.** Therapeutic compound comprising, as an active component, a compound made up in accordance with any one of claims 1 to 9.